# EUROPEAN PATENT SPECIFICATION

(11) **EP 3 233 094 B1**
(45) Date of publication and mention of the grant of the patent: **08.01.2025**
(21) Application number: 15871199.4
(22) Date of filing: 18.12.2015
(51) Int. Cl.: A61K 35/12, A61F 2/02, C12N 15/09, A61K 38/01, A61L 27/36, A61L 27/38, A61K 35/28, A61K 35/30, A61K 35/32, A61K 35/34, A61K 35/35, A61K 35/36, A61K 35/38, A61K 35/44, A61K 35/52, C12N 5/071, A61P 9/00, A61P 43/00

(54) **BIOCOMPATIBLE IMPLANTS COMPRISING ENGINEERED ENDOTHELIAL CELLS**
BIOKOMPATIBLE IMPLANTATE MIT MANIPULIERTEN ENDOTHELZELLEN
IMPLANTS BIOCOMPATIBLES COMPRENANT DES CELLULES ENDOTHÉLIALES MISES AU POINT PAR GÉNIE GÉNÉTIQUE

(30) Priority: 19.12.2014 US 201462094915 P
(43) Date of publication of application: 25.10.2017
(73) Proprietor: Angiocrine Bioscience, Inc., San Diego, CA 92130 (US)
(72) Inventor: GINSBERG, Michael, Daniel, San Diego, California 92130 (US); NOLAN, Daniel, Joseph, Hawthorne, New York 10532 (US); DAVIS, Claude, Geoffrey, Auburn, California 95602 (US)
(74) Representative: Barker Brettell LLP
(86) International application number: PCT/US2015/066782
(87) International publication number: WO 2016/100869

(56) References cited:
- WO-A1-2012/136701
- WO-A2-2008/089448
- US-A1- 2010 168 872
- US-A1- 2013 224 161
- NAKATSU MARTIN N ET AL: "Angiogenic sprouting and capillary lumen formation modeled by human umbilical vein endothelial cells (HUVEC) in fibrin gels: the role of fibroblasts and Angiopoietin-1", MICROVASCULAR RESEARCH, ACADEMIC PRESS, US, vol. 66, no. 2, 1 September 2003 (2003-09-01), pages 102 - 112, XP002453095, ISSN: 0026-2862, DOI: 10.1016/S0026-2862(03)00045-1
- ANDREW C. NEWMAN ET AL: "The requirement for fibroblasts in angiogenesis: fibroblast-derived matrix proteins are essential for endothelial cell lumen formation", MOLECULAR BIOLOGY OF THE CELL, vol. 22, no. 20, 15 October 2011 (2011-10-15), US, pages 3791 - 3800, XP055757219, ISSN: 1059-1524, DOI: 10.1091/mbc.e11-05-0393
- GOODWIN, A.: "In vitro assays of angiogenesis for assessment of angiogenic and anti-angiogenic agents.", MICROVASC RES., vol. 74, no. 2-3, 6 June 2007 (2007-06-06), pages 1 - 20
- KOBAYASHI, K ET AL.: "Role of Etv2-positive cells in the remodeling morphogenesis during vascular development.", GENES CELLS., vol. 18, no. 8, 24 June 2013 (2013-06-24), pages 704 - 721, XP055456548
- SANDLER, VM ET AL.: "Reprogramming human endothelial to hematopoietic cells requires vascular induction.", NATURE, vol. 511, no. 7509, 2 July 2014 (2014-07-02), pages 1 - 43
- GARCIA, MAA ET AL.: "The major histocompatibility complex in transplantation.", JOURNAL OF TRANSPLANTATION., vol. 2012, no. 842141, 20 June 2012 (2012-06-20), pages 1 - 8, XP055387311
- MURO, M ET AL.: "Effect of partial HLA class I match on acute rejection in viral pre-infected human liver allograft recipients.", TRANSPLANTATION., vol. 65, no. 8, 27 April 1998 (1998-04-27), pages 1047 - 1053, XP009503927
- LEIKINA, E ET AL.: "Type I collagen is thermally unstable at body temperature.", PROC NATL ACAD SCI USA., vol. 99, no. 3, 22 January 2002 (2002-01-22), pages 1314 - 1318
- CHOI, YC ET AL.: "Decellularized extracellular matrix derived from porcine adipose tissue as a xenogeneic biomaterial for tissue engineering.", TISSUE ENG PART C METHODS., vol. 18, no. 11, 2 July 2012 (2012-07-02), pages 866 - 876, XP055378331

## Description

### CROSS-REFERENCE TO RELATED APPLICATIONS

This application claims the benefit of U.S. Provisional Patent Application No. 62/094,915, filed December 19, 2014.

### BACKGROUND OF THE INVENTION

Biocompatible implants have been used in a variety of applications, including, for example, to repair or replace damaged or defective tissues within the human body. Some such implants comprise biocompatible scaffold materials that, when implanted surgically into a host subject's body, become populated with the subject's cells and integrate into the subject's tissues. In order to ensure an adequate supply of oxygen and nutrients such implants must, generally, become invested with endothelial cells after surgical implantation, and the endothelial cells must then form functional blood vessels that connect with the subject's existing vasculature. The degree and speed with which endothelial cells infiltrate such implanted scaffolds and form functional blood vessels is thus an important determinant of the ultimate success of such implants. The ability to produce three-dimensional biocompatible scaffolds containing pre-formed blood vessels *in vitro* - i.e. prior to surgical implantation - could significantly increase the speed and efficacy with which such implants integrate into a host subject's tissues *in vivo.* However, prior attempts at generating such implants have met with mixed success. For example, several prior attempts to induce blood vessel formation *in vitro* using collagen gels or Matrigel have failed to result in blood vessel sprouting or the formation of blood vessels having patent lumens surrounded by polarized endothelial cells. (For a discussion of some such prior attempts see Nakatsu et al. (2003) "Angiogenic sprouting and capillary lumen formation modeled by human umbilical vein endothelial cells (HUVEC) in fibrin gels: the role of fibroblasts and angiopoietin-1." Microvascular Research, Vol. 66, pp. 102-112.) Other attempts have involved culturing endothelial cell monolayers on beads coated with extracellular matrix components and then embedding the beads in fibrin gels. Such methods were found to result in the formation of blood vessels with patent lumens only if skin fibroblasts were grown on the top of the gels. Without such fibroblast co-culture the endothelial cells were found to initially form short, narrow cordlike structures which subsequently disintegrated without the formation of blood vessels. See Nakatsu *et al.* (2003). Consistent with these findings, it has been reported that fibroblasts are required for endothelial cell lumen formation. See Newman et al. (2011) "The requirement for fibroblasts in angiogenesis: fibroblast-derived matrix proteins are essential for endothelial cell lumen formation;" Molecular Biology of the Cell, Vol. 22, pp. 3791-3800.

### SUMMARY OF THE INVENTION

The present invention is based, in part, upon the surprising discovery that engineered endothelial cells expressing the adenovirus E4ORF1 protein can form blood vessels having patent lumens inside three-dimensional biocompatible scaffolds *in vitro.* While it was shown previously that E4ORF1+ endothelial cells could form neovessels in Matrigel plugs *in vivo* following co-injection of such cells with liquid Matrigel into the flanks of mice, and that E4ORF1+ endothelial cells could form neo-angiogenic tubes on Matrigel coated culture plates (*See* U.S. Patent No. 8,465,732), to the best of Applicant's knowledge, an ability of such cells to form genuine blood vessels having open lumens within a three-dimensional scaffold *in vitro* has not previously been demonstrated. As described herein, it has now been surprisingly found that true E4ORF1+ blood vessels can form vessels having open lumens inside three-dimensional biocompatible scaffold materials *in vitro* in a period of just a few days - even in the absence of other cell types such as fibroblasts - and that the vessel-containing implants can be cultured and maintained *in vitro* for extended periods of time - up to 6 weeks. Interestingly, it was also found that the blood vessels in these implants could extend beyond the boundaries of the biocompatible scaffolds - creating protruding blood vessels that appeared to float freely in the culture medium surrounding the scaffold material. Building on these discoveries, the present invention provides certain new and improved implants suitable for surgical implantation into subjects, as well as methods for making and using such implants.

Accordingly, in one embodiment the present invention provides an isolated implant suitable for surgical implantation into a subject comprising: (a) a biocompatible porous scaffold material comprising extracellular matrix molecules, collagen, fibrin and/or laminin, or decellularized animal tissue, and (b) blood vessels disposed within the biocompatible scaffold material, wherein the blood vessels comprise E4ORF1+ engineered endothelial cells, and wherein the implants do not comprise fibroblasts, fibroblast-derived angiogenic factors or fibroblast-derived extracellular matrix components.

In another embodiment the present invention provides a method of preparing an implant suitable for surgical implantation into a subject, the method comprising: culturing a population of engineered E4ORF 1+ endothelial cells in contact with a biocompatible porous scaffold material comprising extracellular matrix molecules, collagen, fibrin and/or laminin, or decellularized animal tissue *in vitro* until blood vessels are formed within the biocompatible scaffold material, thereby forming an implant comprising E4ORF 1+ blood vessels.

The biocompatible scaffold materials used in the implants and methods of the invention are, typically, three-dimensional structures - such that vessels can be disposed within the three-dimensional structure. In some such embodiments the implants of the invention contain a network of connected blood vessels, which may comprise capillaries. In some such embodiments the blood vessels have open/patent lumens. In some such embodiments one or more of the blood vessels may protrude beyond the boundaries of the biocompatible scaffold material.

In some embodiments the E4ORF1+ engineered endothelial cells used in the implants and methods of the present invention also express an ETV2 polypeptide (i.e. they are E4ORF1+ ETV2+ endothelial cells). In other such embodiments the E4ORF1+ engineered endothelial cells also express a recombinant ETS family transcription factor (i.e. they are E4ORF1+ETS+).

In some embodiments the engineered E4ORF1+ endothelial cells may be fetal cells, or post-natal cells, or adult cells. In some embodiments the engineered E4ORF1+ endothelial cells used in the implants and methods of the present invention are mammalian endothelial cells. In some embodiments the engineered E4ORF1+ endothelial cells are human endothelial cells. In some embodiments the engineered E4ORF1+ endothelial cells are derived from human umbilical vein endothelial cells (HUVECs).

In some embodiments the engineered E4ORF 1+ endothelial cells used in the implants and methods of the present invention are organ-specific endothelial cells. For example, in some embodiments the engineered endothelial cells may be hematopoietic system-specific endothelial cells, or nervous system-specific endothelial cells, or cardiac-specific endothelial cells, or lung-specific endothelial cells, or liver-specific endothelial cells, or kidney-specific endothelial cells, or muscle-specific endothelial cells, or cartilage-specific endothelial cells, or tendon-specific endothelial cells, or adipose tissue-specific endothelial cells. In some such embodiments the organ-specific endothelial cells used are derived from the organ or tissue into which the implant is to be placed.

In some embodiments the engineered E4ORF1+ endothelial cells used in the implants and methods of the present invention are derived from endothelial cells of the subject into which the implant is to be surgically implanted - i.e. they are autologous endothelial cells. In other embodiments the engineered E4ORF1+ endothelial cells are derived from endothelial cells of a donor of the same species as the subject into which the implant is to be surgically implanted - i.e. they are allogeneic endothelial cells. In some such embodiments the allogeneic donor is tissue matched (or partially matched) with the subject into which the implant is to be surgically implanted. For example, in some embodiments the donor has the same MHC-type (or HLA-type) as the subject into which the implant is to be surgically implanted. In some other embodiments the donor has the an MHC-type (or HLA-type) that is partially matched with that of the subject into which the implant is to be surgically implanted. In yet other embodiments the donor has an MHC-type (or HLA-type) that is different from that of the subject into which the implant is to be surgically implanted.

In some embodiments the biocompatible scaffold materials used in the implants and methods of the present invention of the invention contain one or more additional cell types - in addition to the engineered endothelial cells. In some such embodiments the additional cell types may be genetically modified. In other such embodiments the additional cell types may be naive (i.e. not genetically modified). In some such embodiments the additional cell types may be fetal cells, or post-natal cells, or adult cells. In some embodiments such additional cell types may be stem or progenitor cells. In other embodiments such additional cell types may be differentiated cells. In some embodiments such stem or progenitors cells may be hematopoietic stem cells, bone stem cells, muscle stem cells, neural stem cells, epithelial stem cells, skin stem cells, mesenchymal stem cells, intestinal stem cells, or spermatogonial stem cells. In some embodiments the differentiated cells may be differentiated hematopoietic cells, bone cells, muscle cells, neural cells, pericytes, hair follicle cells, adipose cells, keratinocytes, epithelial cells, skin cells, fibroblasts, intestinal cells, or testicular cells.

In some embodiments the biocompatible scaffold material used in the implants and methods of the present invention within the implants is solid at 4°C. In some embodiments the biocompatible scaffold material is solid at 21°C. In some embodiments the biocompatible scaffold material comprises one or more extracellular matrix molecules, such as collagen or fibrin. In some embodiments the biocompatible scaffold material within the implants comprises decellularized animal tissue, such as decellularized porcine tissue. In some embodiments the biocompatible scaffold material within the implants is not Matrigel. In some embodiments the biocompatible scaffold material within the implants does not comprise hyaluronic acid.

In some embodiments the implants of the invention do not comprise serum. In some embodiments the implants of the invention do not comprise exogenous growth factors. In some embodiments the implants of the invention do not comprise exogenous angiogenic factors. In some embodiments the implants of the invention do not comprise exogenous VEGF. In some embodiments the implants of the invention do not comprise exogenous FGF.

In some embodiments the implants of the invention do not comprise micro-carrier beads. In some embodiments implants of the invention do not comprise micro-carrier beads coated with an extracellular matrix molecule.

These and other embodiments of the invention are described further in the accompanying Examples, Claims, and Drawings sections of this document.

### BRIEF DESCRIPTION OF THE DRAWINGS

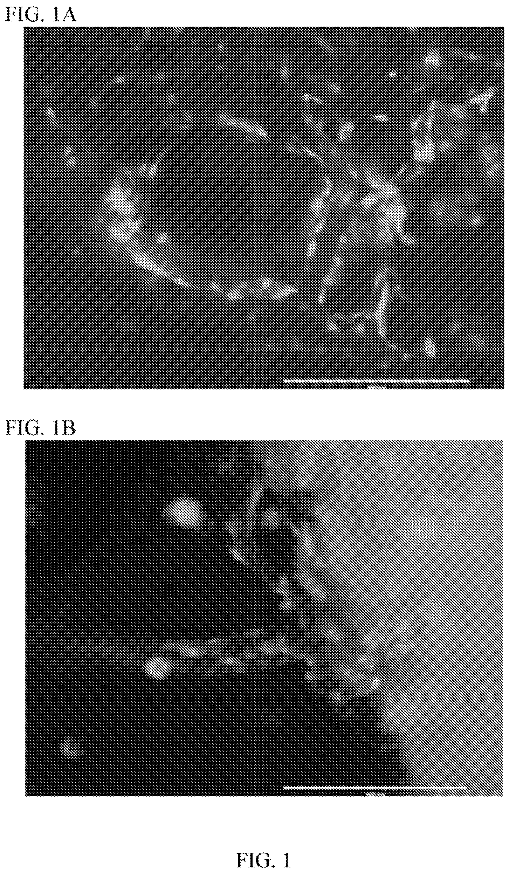

Figure 1A & 1B. Fluorescence microscopy images of implants that had been seeded with engineered endothelial cells expressing E4ORF1 and green fluorescent protein (GFP) and maintained *in vitro* - as described in Example 1. The white bar at the bottom of each image represents 400 microns. The green fluorescence from the GFP expression in the endothelial cells appears as white in the black and white images. Blood vessel structures with open lumens can be seen in both Fig. 1A and Fig. 1B. In Fig. 1B blood vessels can be seen protruding beyond the boundary of the scaffold material.

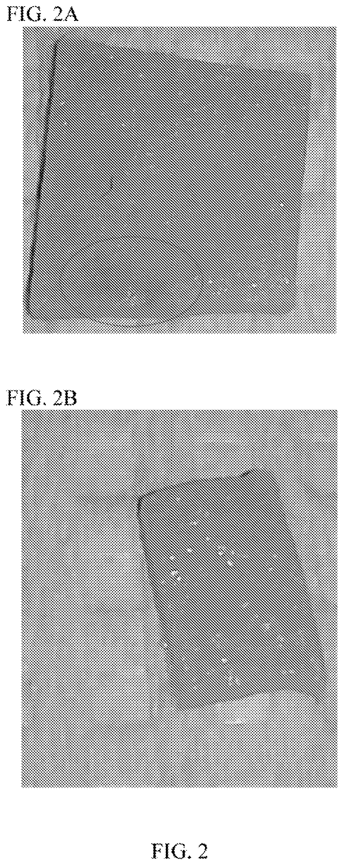

Figure 2A & 2B. Photographs of two implants following removal from the abdomen of a mouse 48 hours following surgical implantation. The "test" implant in Fig. 2A had been seeded with E4ORF1+ engineered endothelial cells and maintained in culture prior to surgical implantation - as described in Examples 1 and 2. Vascularization and blood within the implant can be seen in the circled area of Fig. 2A. The "control" implant in Fig. 2B had not been seeded with endothelial cells prior to surgical implantation and showed no detectable vascularization or blood flow.

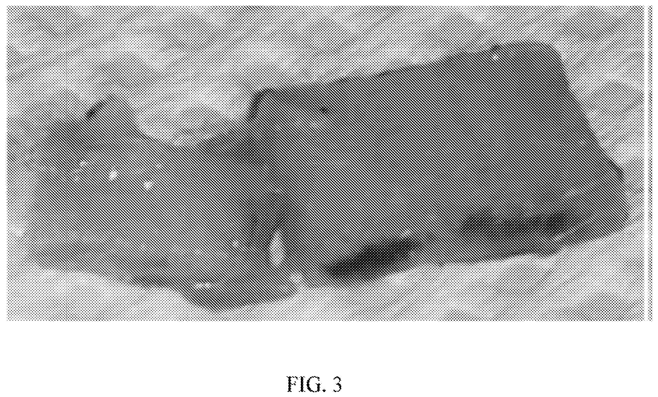

Figure 3. Photographs of two implants following removal from the abdomen of a mouse 4 weeks following surgical implantation. The "test" implant shown on the right had been seeded with E4ORF1+ engineered endothelial cells and maintained in culture prior to implantation - as described in the Examples. Significant vascularization and blood content can be seen in the "test" implant on the right - appearing as dark/black areas on the edge of the implant in this black and white image. The "control" implant on the left had not been seeded with endothelial cells prior to implantation.

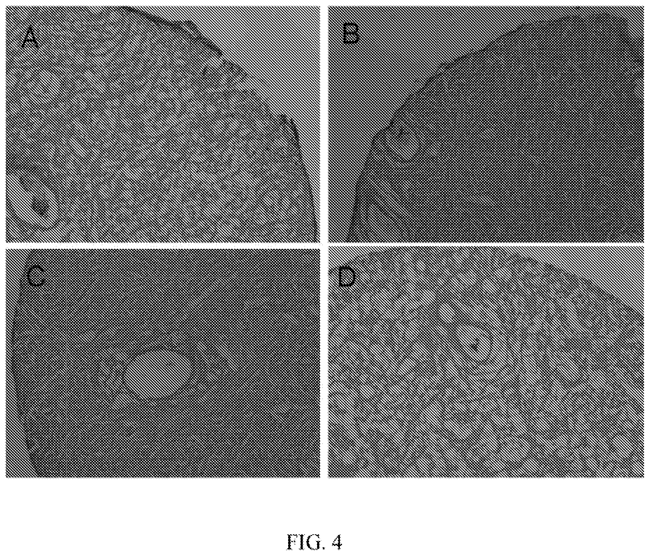

Figure 4A-D. Photomicrographs of histological sections of implants that had been surgically implanted into the abdomen of a mouse and removed 2 weeks after implantation. The "test" implants in Fig. 4A and Fig. 4B had been seeded with E4ORF1+ engineered endothelial cells - as described in the Examples. The "control" implants in Fig. 4C and Fig. 4D had not been seeded with endothelial cells prior to implantation. The "test" implants in Fig. 4A and 4B exhibited increased cellularity as compared to the implants in Fig. 4C and 4D.

### DETAILED DESCRIPTION

The "Summary of the Invention," "Figures," "Brief Description of the Figures," "Examples," and "Claims" sections of this patent disclosure describe some of the main embodiments of the invention. This "Detailed Description" section provides certain additional description relating to the compositions and methods of the present invention, and is intended to be read in conjunction with all other sections of this patent disclosure. Furthermore, and as will be apparent to those in the art, the different embodiments described throughout this patent disclosure can be, and are intended to be, combined in various different ways. Such combinations of the specific embodiments described herein are intended to fall within the scope of the present invention

Certain definitions are provided below. Other terms are either defined elsewhere in this patent disclosure, have a meaning that is clear from the context in which they are used, or are used in accordance with their usual meaning in the art.

### Definitions

As used herein, the terms "about" and "approximately," when used in relation to numerical values, mean within + or - 20% of the stated value.

The term "culturing" as used herein, refers to the propagation of cells on or in media of various kinds. "Co-culturing" refers to the propagation of two or more distinct types of cells on or in media of various kinds, for instance, in some embodiments, endothelial cells and stem or progenitor cells may be co-cultured.

As used herein the term "effective amount" refers to an amount of a specified agent or cell population (e.g. an E4ORF1 polypeptide, a nucleic acid molecule encoding an E4ORF1 polypeptide, or a population of E4ORF1+ engineered endothelial cells), as described herein, that is sufficient to achieve a detectable effect on one or more of the outcomes described herein. For example, in the case of expression of E4ORF1 in endothelial cells an effective amount of a nucleic acid molecule (e.g. in a vector) to be introduced/delivered to the endothelial cells may be one that results in a detectable increase in the endothelial cells survival or proliferation as compared to that of any suitable control (e.g. E4ORF1⁻endothelial cells). In the case of introduction of nucleic acid molecules encoding E4ORF1 into endothelial cells, an effective amount of the nucleic acid molecule (e.g. in a vector) may be one that results in a detectable increase in the endothelial cells survival or proliferation as compared to that of any suitable control (e.g. E4ORF1⁻ cells). In the case of methods that involve administering E4ORF1⁺ endothelial cells to a subject, an effective amount may be one that results in a detectable improvement of one or more desired biological or therapeutic indicators, (such as, for example, improved endothelial/vascular regeneration, improved angiogenesis, improved survival or engraftment of an implant, etc.), as compared to that of any suitable control (e.g. E4ORF1⁻ endothelial cells). An appropriate "effective amount" in any individual case may be determined empirically, for example using standard techniques known in the art, such as dose escalation studies, and may be determined taking into account such factors as the planned use, the planned mode of delivery/administration, desired frequency of delivery/administration, etc. Furthermore, an "effective amount" may be determined using assays such as those described in the Examples section of this patent disclosure to assess the formation of blood vessels in implants and/or toassess the integration of an implant into a tissue *in vivo.*

The term "engineered" when used in relation to cells herein refers to cells that have been engineered by man to result in the recited phenotype (e.g. E4ORF1⁺ or ETV2⁺ or expressing a recombinant ETS transcription factor), or to express a recited nucleic acid molecule or polypeptide. The term "engineered cells" is not intended to encompass naturally occurring cells, but is, instead, intended to encompass, for example, cells that comprise a recombinant nucleic acid molecule, or cells that have otherwise been altered artificially (e.g. by genetic modification - as defined below), for example so that they express a polypeptide that they would not otherwise express, or so that they express a polypeptide at substantially higher levels than that observed in non-engineered endothelial cells.

"Genetic modification" or "gene-modified" or "genetically modified" refers to any addition, deletion or disruption of or to a cell's normal nucleotide sequences. For example, in some embodiments, the endothelial cells described have been genetically modified such that they contain a nucleic acid molecule that encodes an adenovirus E4ORF1 polypeptide, and/or a nucleic acid molecule that encodes any of the other specific polypeptides described herein (e.g. ETS transcription factor polypeptides, such as ETV2 polypeptides). Similarly, in some embodiments the endothelial cells described herein, or any of the other cell types described herein (such as stem or progenitor cells or neural cells, muscle cells, pericytes, epithelial cells, adipose cells, fibroblasts, keratinocytes, monocytes, neutrophils, lymphocytes, T-cells, B-cells, or hair follicle stem cells. ) may also comprise one or more other genetic modifications - as desired. The term "genetic modification" encompasses use of a gene delivery vehicle and includes, but is not limited to, transduction (viral mediated transfer of nucleic acid to a recipient, either *in vivo* or *in vitro*), transfection (uptake by cells of isolated nucleic acid), liposome mediated transfer and others means well known in the art..

The term "naive" or "wild-type" when used in relation to cells herein refers to cells that have not been genetically modified (as that term is defined above) or that are not "engineered" cells (as that term is defined above). For example, endothelial cells or other cell types that have been obtained from a subject and that have not been genetically modified by man are considered "naive" or "wild-type" cells.

As used herein the phrase "isolated implant" refers to an implant that is not inside the body of a subject, but that is instead *ex vivo* / *in vitro.* For example, an implant *in vitro* in a culture medium is considered to be an "isolated implant." Typically an "isolated" implant is one that was created *in vitro* as opposed to *in vivo,* and that has not yet been implanted or incubated *in vivo.* Thus, typically, an isolated implant refers to an implant created *in vitro* that, while suitable for surgical implantation, has not yet been surgically implanted into a subject. All of the embodiments of the invention that refer to "implants" typically involve "isolated implants."

As used herein, the term "recombinant" refers to nucleic acid molecules that are generated by man (including by a machine) using methods of molecular biology and genetic engineering (such as molecular cloning), and that comprise nucleotide sequences that would not otherwise exist in nature. Thus, recombinant nucleic acid molecules are to be distinguished from nucleic acid molecules that exist in nature - for example in the genome of an organism. A nucleic acid molecule that comprises a complementary DNA or "cDNA" copy of an mRNA sequence, without any intervening intronic sequences such as would be found in the corresponding genomic DNA sequence, would thus be considered a recombinant nucleic acid molecule. By way of example, a recombinant E4ORF1 nucleic acid molecule might comprise an E4ORF1 coding sequence operatively linked to a promoter and/or other genetic elements with which that coding sequence is not ordinarily associated in a naturally-occurring adenovirus genome. Similarly, a recombinant ETV2 nucleic acid molecule might comprise an ETV2 cDNA sequence (i.e. a sequence that does not exist in nature in the genome of an organism), and/or may comprise ETV2-coding sequences operatively linked to a promoter and/or other genetic elements with which that coding sequence is not ordinarily associated in the genome of an organism.

The terms "subject" and "patient" are used herein interchangeably herein and refer to, except where indicated, mammals such as humans and non-human primates, as well as rabbits, rats, mice, goats, pigs, and other mammalian species.

The phrase "substantially pure" as used herein in relation to a cell population refers to a population of cells of a specified type (e.g. as determined by expression of one or more specified cell markers, morphological characteristics, or functional characteristics), or of specified types (plural) in embodiments where two or more different cell types are used together, that is at least about 50%, preferably at least about 75-80%, more preferably at least about 85-90%, and most preferably at least about 95% of the cells making up the total cell population. Thus, a "substantially pure cell population" refers to a population of cells that contain fewer than about 50%, preferably fewer than about 20-25%, more preferably fewer than about 10-15%, and most preferably fewer than about 5% of cells that are not of the specified type or types.

### Nucleic Acid Molecules and Polypeptides

The adenoviral early 4 (E4) region contains at least 6 open reading frames (E4ORFs). The entire E4 region has been shown previously to regulate angiogenesis and promote survival of endothelial cells (see Zhang et al. (2004), J. Biol. Chem. 279(12): 11760-66). It has also been shown previously that, within the entire E4 region, it is the E4ORF1 sequence that is responsible for these biological effects in endothelial cells. *See* U.S. Patent No. 8,465,732. *See also* Seandel et al. (2008), "Generation of a functional and durable vascular niche by the adenoviral E4ORF1 gene," PNAS, 105(49):19288-93. Several of the embodiments of the present invention described herein involve engineered endothelial cells that are E4ORF1+ - i.e. that express an E4ORF1 polypeptide. Similarly, several of the embodiments of the present invention described herein involve engineered endothelial cells that are ETV2+ or ETS+. Such cells contain express an ETV2 polypeptide or an ETS family transcription factor polypeptide, respectively. All of these polypeptides (E4ORF1, ETV2, ETS) are referred to collectively herein as "polypeptides of the invention".

The "polypeptides of the invention" are encoded by nucleic acid molecules. Thus, in some embodiments the present invention involves nucleic acid molecules that encode an adenovirus E4ORF1 polypeptide, nucleic acid molecules that encode an ETS transcription factor polypeptide, and/or nucleic acid molecules that encode an ETV2 transcription factor polypeptide. Such nucleic acid molecules are referred to collectively herein as "nucleic acid molecules of the invention."

The polypeptides of the invention and the nucleic acid molecules of the invention may have amino acid sequences or nucleotide sequences that are specified herein or known in the art, or may have amino acid or nucleotide sequences that are variants, derivatives, mutants, or fragments of such amino acid or nucleotide sequences - provided that such a variants, derivatives, mutants, or fragments have, or encode a polypeptide that has, one or more of the functional properties described herein, or one or more of the properties required for the uses described herein, or does not prevent or block one or more of the properties or uses described herein (which include, but are not limited to, an ability to promote the survival of endothelial cells *in vitro,* and an ability of endothelial cells to form blood vessels in an implant *in vitro*).

In those embodiments involving ETS transcription factor polypeptides, such as ETV2 polypeptides, the polypeptide may be any mammalian ETS transcription factor polypeptide, such as a human, non-human primate, rabbit, rat, mouse, goat, or pig polypeptide. In some preferred embodiments the polypeptide may be a human polypeptide. Amino acid sequences of such polypeptides, and nucleic acid sequences that encode such polypeptides, are well known in the art and available in well-known publicly available databases, such as the Genbank database.

In those embodiments involving adenovirus E4ORF1 polypeptides, the polypeptide sequence used may be from any suitable adenovirus type or strain, such as human adenovirus type 2, 3, 5, 7, 9, 11, 12, 14, 34, 35, 46, 50, or 52. In some preferred embodiments the polypeptide sequence used is from human adenovirus type 5. Amino acid sequences of such adenovirus polypeptides, and nucleic acid sequences that encode such polypeptides, are well known in the art and available in well-known publicly available databases, such as the Genbank database. For example, suitable sequences include the following: human adenovirus 9 (Genbank Accession No. CAI05991), human adenovirus 7 (Genbank Accession No. AAR89977), human adenovirus 46 (Genbank Accession No. AAX70946), human adenovirus 52 (Genbank Accession No. ABK35065), human adenovirus 34 (Genbank Accession No. AAW33508), human adenovirus 14 (Genbank Accession No. AAW33146), human adenovirus 50 (Genbank Accession No. AAW33554), human adenovirus 2 (Genbank Accession No. AP.sub.--000196), human adenovirus 12 (Genbank Accession No. AP.sub.-000141), human adenovirus 35 (Genbank Accession No. AP.sub.--000607), human adenovirus 7 (Genbank Accession No. AP.sub.--000570), human adenovirus 1 (Genbank Accession No. AP.sub.--000533), human adenovirus 11 (Genbank Accession No. AP.sub.-000474), human adenovirus 3 (Genbank Accession No. ABB 17792), and human adenovirus type 5 (Genbank accession number D12587).

In some embodiments, the polypeptides and nucleic acid molecules of the invention have the same amino acid or nucleotide sequences as those specifically recited herein or known in the art (for example in public sequence databases, such as the Genbank database). In some embodiments the polypeptides and nucleic acid molecules of the invention may have amino acid or nucleotide sequences that are variants, derivatives, mutants, or fragments of such sequences, for example variants, derivatives, mutants, or fragments having greater than 85% sequence identity to such sequences. In some embodiments, the variants, derivatives, mutants, or fragments have about an 85% identity to the known sequence, or about an 88%, 89%, 90%, 91%, 92%, 93%, 94%, 95%, 96%, 97%, 98%, or 99% sequence identity to the known sequence. In some embodiments, a variant, derivative, mutant, or fragment of a known nucleotide sequence is used that varies in length by about 50 nucleotides, or about 45 nucleotides, or about 40 nucleotides, or about 35 nucleotides, or about 30 nucleotides, or about 28 nucleotides, 26 nucleotides, 24 nucleotides, 22 nucleotides, 20 nucleotides, 18 nucleotides, 16 nucleotides, 14 nucleotides, 12 nucleotides, 10 nucleotides, 9 nucleotides, 8 nucleotides, 7 nucleotides, 6 nucleotides, 5 nucleotides, 4 nucleotides, 3 nucleotides, 2 nucleotides, or 1 nucleotide relative to the known nucleotide sequence. In some embodiments, a variant, derivative, mutant, or fragment of a known amino sequence is used that varies in length about 50 amino acids, or about 45 amino acids, or about 40 amino acids, or about 35 amino acids, or about 30 amino acids, or about 28 amino acids, 26 amino acids, 24 amino acids, 22 amino acids, 20 amino acids, 18 amino acids, 16 amino acids, 14 amino acids, 12 amino acids, 10 amino acids, 9 amino acids, 8 amino acids, 7 amino acids, 6 amino acids, 5 amino acids, 4 amino acids, 3 amino acids, 2 amino acids, or 1 amino acid relative to the known amino acid sequence.

In those embodiments where an E4ORF1 nucleic acid or amino acid sequence is used, in some embodiments such sequences are used without other sequences from the adenovirus E4 region - for example not in the context of the nucleotide sequence of the entire E4 region or not together with other polypeptides encoded by the E4 region. However, in some other embodiments such sequences may be used in conjunction with one or more other nucleic acid or amino acid sequences from the E4 region, such as E4ORF2, E4ORF3, E4ORF4, or E4ORF5 sequences, or variants, mutants or fragments thereof. For example, although E4ORF1 sequences can be used in constructs (such as a viral vectors) that contain other sequences, genes, or coding regions (such as promoters, marker genes, antibiotic resistance genes, and the like), in certain embodiments, the E4ORF 1 sequences are used in constructs that do not contain the entire E4 region, or that do not contain other ORFs from the entire E4 region, such as E4ORF2, E4ORF3, E4ORF4, and/or E4ORF5.

The nucleic acid molecules of the invention can be used in constructs that contain various other nucleic acid sequences, genes, or coding regions, depending on the desired use, for example, antibiotic resistance genes, reporter genes or expression tags (such as, for example nucleotides sequences encoding GFP), or any other nucleotide sequences or genes that might be desirable. The polypeptides of the invention can be expressed alone or as part of fusion proteins.

In some embodiments nucleic acid molecules of the invention can be under the control of one or more promoters to allow for expression. Any promoter able to drive expression of the nucleic acid sequences in the desired cell type can be used. Examples of suitable promoters include, but are not limited to, the CMV, SV40, RSV, HIV-Ltr, and MML promoters. The promoter can also be a promoter from the adenovirus genome, or a variant thereof. For example, where E4ORF1 is used, the promoter can be the promoter used to drive expression of corresponding genes in an adenovirus.

In some embodiments, nucleic acid molecules of the invention can be placed under the control of an inducible promoter, so that expression of the nucleic acid sequences can be turned on or off as desired. Any suitable inducible expression system can be used, such as, for example, a tetracycline inducible expression system, or a hormone inducible expression system. For example, the nucleic acid molecules of the invention can be expressed while they are needed and then switched off when the desired outcome has been achieved, for example when there has been sufficient growth or proliferation of the endothelial cells. The ability to turn on or turn off expression could be particularly useful for *in vivo* applications.

The nucleic acid molecules of the invention may comprise naturally occurring nucleotides, synthetic nucleotides, or a combination thereof. For example, in some embodiments the nucleic acid molecules of the invention can comprise RNA, such as synthetic modified RNA that is stable within cells and can be used to direct protein expression/production directly within cells. In other embodiments the nucleic acid molecules of the invention can comprise DNA. In embodiments where DNA is used, the DNA sequences may be operably linked to one or more suitable promoters and/or regulatory elements to allow (and/or facilitate, enhance, or regulate) expression within cells, and may be present in one or more suitable vectors or constructs. The nucleic acid molecules of the invention can be introduced into endothelial cells in the same nucleic acid construct or they can be introduced in separate nucleic acid constructs.

The nucleic acid molecules of the invention can be introduced into endothelial cells using any suitable system known in the art, including, but not limited to, transfection techniques and viral-mediated transduction techniques. Transfection methods that can be used in accordance with the present invention include, but are not limited to, liposome-mediated transfection, polybrene-mediated transfection, DEAE dextran-mediated transfection, electroporation, calcium phosphate precipitation, microinjection, and microparticle bombardment. Viral-mediated transduction methods that can be used include, but are not limited to, lentivirus-mediated transduction, adenovirus-mediated transduction, retrovirus-mediated transduction, adeno-associated virus-mediated transduction and herpes virus-mediated transduction.

The present invention also provides vectors, including expression vectors that contain nucleic acid molecules of the invention. For example, in one embodiment, the present invention provides an expression vector comprising a nucleotide sequence encoding an ETS transcription factor polypeptide and a nucleotide sequence encoding an E4ORF1 polypeptide. In some such embodiments the ETS transcription factor is ETV2. In some such embodiments the expression vector is a lentivirus vector. In some embodiments the nucleotide sequence encoding the ETS transcription factor polypeptide and the nucleotide sequence encoding the E4ORF 1 polypeptide are under the control of separate promoters. In some embodiments the nucleotide sequence encoding the ETS transcription factor polypeptide and the nucleotide sequence encoding the E4ORF 1 polypeptide are under the control of the same promoter, for example with an internal ribosome entry site sequence (IRES) between the ETS and E4ORF1 sequences.

In some embodiments a peptidomimetic may be used. A peptidomimetic is a small protein-like chain designed to mimic a polypeptide. Such a molecule could be designed to mimic any of the polypeptides of the invention (e.g. an ETV2 or E4ORF1 polypeptide). Various different ways of modifying a peptide to create a peptidomimetic or otherwise designing a peptidomimetic are known in the art and can be used to create a peptidomimetic of one of the polypeptides of the invention.

The handling, manipulation, and expression of the polypeptides and nucleic acid molecules of the invention may be performed using conventional techniques of molecular biology and cell biology. Such techniques are well known in the art. For example, one may refer to the teachings of Sambrook, Fritsch and Maniatis eds., "Molecular Cloning A Laboratory Manual, 2nd Ed., Cold Springs Harbor Laboratory Press, 1989); the series Methods of Enzymology (Academic Press, Inc.), or any other standard texts for guidance on suitable techniques to use in handling, manipulating, and expressing nucleotide and/or amino acid sequences. Additional aspects relevant to the handling or expression of E4ORF1 sequences are described in U.S. Patent No. 8,465,732.

### Engineered Endothelial Cells

In some embodiments the present invention provides "engineered endothelial cells."

In some embodiments the engineered endothelial cells are E4ORF1+ engineered endothelial cells. In some embodiments the engineered endothelial cells are E4ORF1+ETV2+ engineered endothelial cells. In some embodiments the engineered endothelial cells are E4ORF1+ETS+ engineered endothelial cells.

In other embodiments the engineered endothelial cells express one or more markers. Indeed, it should be noted that in all of the embodiments described herein that involve E4ORF1+ engineered endothelial cells, as an alternative to the E4ORF1+ cells, endothelial cells can be used that have been engineered (for example by transfection or transduction with a nucleic acid molecule, or by treatment with any other agent, such as a protein or chemical agent) such that they express one or more of the following markers, or express elevated levels of one or more of the following markers, such as 2-fold, 3-fold, 4-fold, 5-fold, 6-fold, 7-fold, 8-fold, 9-fold, 10-fold, 20-fold, 30-fold, 40-fold, or 50-fold higher levels of one or more of the following markers, as compared to non-engineered endothelial cells (e.g. as compared to naive endothelial cells or as compared to endothelial cells that have not been so-engineered but have otherwise been treated comparably). The markers are: Integrin alpha 11, Matrilin 2, TIMP metallopeptidase inhibitor 3, Delta-like 4, Elastin, Smoothelin-like 2, Syndecan 1, Calponin 3 acidic, Protein C (inactivator of coagulation factors Va and Villa), Growth differentiation factor 3, Gap junction protein alpha 4 37kDa, ADAM metallopeptidase with thrombospondin type 1 motif 18, Interleukin 33, Leptin receptor, Sulfatase 1, Ephrin-A1, Dipeptidyl-peptidase 4, Collagen, type VIII, alpha 1, Chemokine (C-C motif) ligand 2, Cartilage acidic protein 1, Cysteinyl leukotriene receptor 2, Fibroblast growth factor 2 (basic), Laminin beta 3, CD82 molecule, Insulin receptor, Ephrin-B1, Chemokine (C-C motif) receptor-like 1, Gap junction protein, alpha 4, 37kDa, and Fibulin 2. The above listed markers, and their corresponding gene symbols, are also illustrated in Table 1, below.

**Table 1: Markers Up-regulated in Engineered E4ORF1+ Endothelial Cells**

| **Gene Name** | **Gene Symbol** | **Fold increase in expression*** |
|---|---|---|
| Integrin, alpha 11 | ITGA11 | 3.84 |
| Matrilin 2 | MATN2 | 7.97 |
| TIMP metallopeptidase inhibitor 3 | TiMP3 | 6.23 |
| Delta-like 4 (Drosophila) | DLL4 | 2.24 |
| Elastin | ELN | 91.37 |
| Smoothelin-like 2 | SMTNL2 | 9.52 |
| Syndecan 1 | SDC1 | 4.23 |
| Calponin 3, acidic | CNN3 | 1.55 |
| Protein C (inactivator of coagulation factors Va and Villa) | PROC | 10.48 |
| Growth differentiation factor 3 | GDF3 | 7.62 |
| Gap junction protein, alpha 4, 37kDa | GJA4 | 4.15 |
| ADAM metallopeptidase with thrombospondin type 1 motif 18 | ADAMT18 | 2.93 |
| Interleukin 33 | IL33 | 6.14 |
| Leptin receptor | LEPR | 5.13 |
| Sulfatase 1 | SULF1 | 3.36 |
| Ephrin-A1 | EFNA1 | 2.03 |
| Dipeptidyl-peptidase 4 | DPP4 | 4.62 |
| Collagen, type VIII, alpha 1 | COLA1 | 4.50 |
| Chemokine (C-C motif) ligand 2 | CCL2 | 3.38 |
| Cartilage acidic protein 1 | CRTAC1 | 10.41 |
| Cysteinyl leukotriene receptor 2 | CYSLTR2 | 5.42 |
| Fibroblast growth factor 2 (basic) | FGF2 | 4.81 |
| Laminin, beta 3 | LAMB3 | 2.87 |
| CD82 molecule | CD82 | 2.18 |
| Insulin receptor | INSR | 2.36 |
| Ephrin-B1 | EFNB1 | 2.44 |
| Chemokine (C-C motif) receptor-like 1 | CCRL1 | 2.69 |
| Gap junction protein, alpha 4, 37kDa | GJA4 | 4.15 |
| Fibulin 2 | FBLN2 | 10.13 |

| | | |
|---|---|---|
| * fold-increase in expression in passage 12 (P12) E4ORF1-expressing HUVECs as compared to (P12) non-E4ORF1-expressing HUVECs, as determined by RNA-Seq. | | |

In some such embodiments the engineered endothelial cells express, or express elevated levels of, 2, 3, 4, 5, 6, 7, 8, 9, 10, 11, 12, 13, 14, 15, 16, 17, 18, 19, 20, 21, 22, 23, 24, 25, 26, 27, 28, or all 29 of the above markers. In some such embodiments the engineered endothelial cells express, or express elevated levels of, elastin. In some such embodiments the engineered endothelial cells express, or express elevated levels of, elastin, protein C, cartilage acidic protein 1, and fibulin 2. In some such embodiments the engineered endothelial cells express, or express elevated levels of, elastin, protein C, cartilage acidic protein 1, fibulin 2, matrilin 2, smoothelin-like 2, growth-differentiation factor 3, interleukin 33, leptin receptor and cysteinyl leukotriene receptor 2.

The engineered endothelial cells can be derived from any suitable source of endothelial cells known in the art. In some embodiments the endothelial cells are primary endothelial cells. In some embodiments the engineered endothelial cells are mammalian cells, such as human or non-human primate cells, or rabbit, rat, mouse, goat, pig, or other mammalian cells. In some embodiments the endothelial cells are primary human endothelial cells. In some embodiments the endothelial cells are umbilical vein endothelial cells (UVECs), such as human umbilical vein endothelial cells (HUVECs). In some embodiments the endothelial cells are naive endothelial cells. In some embodiments the endothelial cells are genetically modified endothelial cells. In some embodiments the engineered endothelial cells can be derived from stem cells or progenitor cells. For example, in some embodiments the engineered endothelial cells can be derived from pluripotent stem cells, such as induced pluripotent stem cells (iPS cells) or embryonic stem cells (ES cells). In some embodiments the engineered endothelial cells can be derived from endothelial progenitor cells. In some embodiments the engineered endothelial cells can be derived from a differentiated non-endothelial cell type using a reprogramming method, such as a direct reprogramming method or a method in which a differentiated cell is reprogrammed into a less differentiated cell type (such as a pluripotent or multipotent cell type) prior to subsequently differentiating the less differentiated cell into an endothelial cell. In some embodiments the engineered endothelial cells are organ-specific endothelial cells. For example, in some embodiments the engineered endothelial cells may be hematopoietic system-specific endothelial cells, or nervous system-specific endothelial cells, or cardiac-specific endothelial cells, or lung-specific endothelial cells, or liver-specific endothelial cells, or kidney-specific endothelial cells, or muscle-specific endothelial cells, or cartilage-specific endothelial cells, or adipose tissue-specific endothelial cells. In some such embodiments the organ-specific endothelial cells used are derived from the organ or tissue into which the implant is to be placed. For example, in some embodiments if the implant is to be implanted into lung tissue, or used to treat a lung defect, the endothelial cells used may lung-specific endothelial cells. Similarly, if the implant is to be implanted into liver tissue, or used to treat a liver defect, the endothelial cells used may be liver-specific endothelial cells. Other suitable endothelial cells that can be used include those described previously as being suitable for E4ORF 1-expression in U. S. Patent No. 8,465,732.

In some embodiments the engineered endothelial cells are genetically-modified such that they comprise one or more genetic modifications in addition to and apart from the expression of any of the specific molecules or markers described herein (e.g. E4ORF 1). For example, such cells may comprise a corrected version of a gene known to be involved in, or suspected of being involved in, a disease or disorder that affects endothelial cells, or any other gene, such as a therapeutically useful gene, that it may be desired to provide in endothelial cells or administer or deliver using engineered endothelial cells.

The engineered endothelial cells of the invention may exist in, or be provided in, various forms. For example, in some embodiments the engineered endothelial cells may comprise a population of cells, such as an isolated population of cells. In some embodiments the engineered endothelial cells may comprise a population of cells *in vitro* - for example to be added to an implant, or comprised within an implant. In some embodiments the engineered endothelial cells may comprise a substantially pure population of cells. Similarly in some embodiments the isolated implants of the invention may comprise a substantially pure population of endothelial cells. For example, in some embodiments at least about 50%, preferably at least about 75-80%, more preferably at least about 85-90%, and most preferably at least about 95% of the cells making up a total cell population will be engineered endothelial cells of the invention. In some embodiments the engineered endothelial cells may be provided in the form of a composition containing the engineered cells and one or more additional components. For example, in some embodiments the present invention may provide a composition comprising a population of engineered endothelial cells as described herein together with a carrier solution, such as a physiological saline solution, cell suspension medium, cell culture medium, or the like. In some embodiments such compositions may be therapeutic compositions - comprising a population of engineered endothelial cells and a carrier solution that is suitable for administration to a subject, such as a human subject. Other therapeutically acceptable agents can be included if desired. One of ordinary skill in the art can readily select suitable agents to be included in the therapeutic compositions depending on the intended use.

In some embodiments the engineered endothelial cells of the invention are mitotically inactivated prior to use (e.g. therapeutic use) such that they cannot replicate. This can be achieved, for example, by using a chemical agent such as mitomycin C or by irradiating the engineered endothelial cells.

### Biocompatible Scaffolds

In certain embodiments the biocompatible scaffold used in accordance with the present invention can be, or can comprise, consist essentially of, or consist of, of any material that is biocompatible, is capable of being infiltrated by cells (e.g. comprising a porous structure), and is suitable for surgical implantation into a living subject. Examples of such scaffolds include, but are not limited to, those that comprise, consist of, or consist essentially of de-cellularized animal tissue (such as de-cellularized porcine tissue, e.g. XenMatrix available from Bard, Inc.), or one or more extracellular matrix ("ECM") components such as collagen, laminin, and/or fibrin. In some embodiments the biocompatible scaffold comprises at least about 5%, 10%, 20%, 30%, 40%, 50%, 60%, 70% , 80%, 90% or 95% collagen. In some embodiments the biocompatible scaffold comprises at least about 5%, 10%, 20%, 30%, 40%, 50%, 60%, 70% , 80%, 90% or 95% laminin. In some embodiments the biocompatible scaffold comprises at least about 5%, 10%, 20%, 30%, 40%, 50%, 60%, 70% , 80%, 90% or 95% fibrin. In some embodiments the biocompatible scaffold is one that is solid at 4°C and/or at room temperature (around 21°C), or both. In some embodiments the biocompatible scaffold does not comprise hyaluronic acid. In some embodiments the biocompatible scaffold does not comprise more than about 5%, 4%, 3%, 2%, 1%, or 0.5% hyaluronic acid. In some embodiments the biocompatible scaffold does not comprise Matrigel. In some embodiments the biocompatible scaffold material may be selected depending on the tissue location into which it is to be implanted, for example based on its biomechanical properties or any other biological properties.

### Implants

The implants described herein comprise a biocompatible scaffold material and engineered endothelial cells. In some embodiments the biocompatible scaffold has a three-dimensional structure and the engineered endothelial cells form blood vessels within the implant.

In some embodiments the implants may also comprise other cell types - in addition to endothelial cells. In some embodiments such additional cell types may be stem or progenitor cells, such as embryonic stem (ES) cells, induced pluripotent stem cells (iPSCs), hematopoietic stem cells, bone stem cells, muscle stem cells, neural stem cells, epithelial stem cells, skin stem cells, mesenchymal stem cells, intestinal stem cells, spermatogonial stem cells, and the like. In some embodiments such additional cell types may differentiated cells such as hematopoietic cells, bone cells, muscle cells, neural cells, epithelial cells, skin cells, intestinal cells, testicular cells, and the like. In some embodiments such additional cell types may be genetically modified cells. In some embodiments such additional cell types may be naive (i.e. non-genetially modified) cells. Other examples of cells that can be provided or used together with the engineered endothelial cells of the invention are provided in U.S. Patent No. 8,465,732.

In some embodiments the implant can be of any suitable size and/or shape depending on the intended use of the implant. For example, if an implant is intended for replacement or repair of a specific tissue defect, the implant can be configured such that it has a size and shape suitable for insertion into that tissue defect.

### Culture Methods & Methods of Making Implants

Methods of culturing cells are well known in the art and any suitable cell culture methods can be used. For example, the engineered endothelial cells of the invention, or implants containing such cells, can be cultured using methods known to be useful for culturing other endothelial cells, or, methods known to be useful for culturing E4ORF 1+ endothelial cells, for example as described in U.S. Patent No. 8,465,732. In some embodiments the engineered endothelial cells of the invention, or implants containing such cells, can be cultured in the absence of serum, or in the absence of exogenous growth factors, or in the absence of both serum and exogenous growth factors, or in the absence of exogenous angiogenic factors.

The engineered endothelial cells of the invention can also be cryopreserved. Various methods for cell culture and cell cryopreservation are known to those skilled in the art, such as the methods described in Culture of Animal Cells: A Manual of Basic Technique, 4th Edition (2000) by R. Ian Freshney ("Freshney").

The implants of the present invention can be made by any suitable method known in the art. For example, in one embodiment the present invention provides a method for preparing an implant as described herein, comprising contacting a biocompatible scaffold with a population of engineered endothelial cells *in vitro.* In some embodiments, the initial contacting step may comprise placing a biocompatible scaffold material into, or on top of, a culture of engineered endothelial cells. In some embodiments, the initial contacting step may comprise placing engineered endothelial cells on to or into a biocompatible scaffold material. During the initial contacting step, and thereafter, the engineered endothelial cells, the biocompatible scaffold, and the resulting implant, can be maintained under culture conditions that are known to be suitable for the culture of endothelial cells, such as those described herein. The biocompatible scaffold may be incubated with the population of engineered endothelial cells for sufficient time for the endothelial cells to infiltrate the biocompatible scaffold, or for sufficient time for the endothelial cells to form blood vessels within biocompatible scaffold, as desired. For example, in some embodiments, the biocompatible scaffold may be incubated with the population of engineered endothelial cells for about 1 day (24 hours), 2 days (48 hours), 3 days, 4 days, 5 days, 6 days, or 7 days (1 week), or for about 2 weeks, 3 weeks, 4 weeks, 5 weeks, or 6 weeks, or more, prior to use of the implant, for example for implantation into a subject.

### Subjects & Methods of Treatment

In some embodiments, the subjects into which the implants of the invention may be implanted are subjects of any animal species in which it may be desired to use an implant to aid in the treatment of a tissue defect. In some embodiments the subject is a mammal, for example a mammal selected from the group consisting of primates (such as humans and monkeys), rodents, (such as mice, rats and rabbits), ovine species (such as sheep and goats), bovine species (such as cows), porcine species, equine species, feline species and canine species. In some embodiments the subject is a human.

The implants of the invention may be used to treat a tissue defect in a subject in need thereof. Such subjects may have a "tissue defect." As used herein the term "tissue defect" is intended to encompass a variety of types of conditions and types of tissue damage, tissue injury, or tissue dysfunction, including, but not limited to, that caused by traumatic injury, ageing, degenerative disease, genetic disorders, infectious disease, autoimmune disease, or cancer. The methods of treatment provided herein may provide, or be aimed at achieving, replacement, reconstruction, regeneration, repair, or supplementation of a tissue defect in the subject. For example, in one embodiment an implant as described herein can be used to treat diabetes in a subject (e.g. by replacement or supplementation of defective insulin-secreting tissue), or treat neuropathy. In another embodiment an implant as described herein can be used to treat neuropathy, for example by placing an implant in the vicinity of a damaged nerve, e.g. a nerve having a damaged, defective or absent myelin sheath, where such implant may facilitate repair of the nerve, including its myelin sheath.

The implants of the present invention can be surgically implanted into a subject in need thereof using standard surgical methods or techniques known in the art. For example, an implant according to the present invention may be surgically implanted at the site of a tissue defect, or close to the site of a tissue defect, as needed.

In some embodiments the implants of the invention contain engineered endothelial cells that are derived from the same subject into which the implant is to be implanted - i.e. autologous cells. In other embodiments the implants of the invention contain engineered endothelial cells that are derived from an allogeneic donor subject. In some such embodiments the allogeneic donor has a tissue match with the subject into which the implant will be placed (e.g. HLA matched, or MHC matched). In some such embodiments the allogenic donor is mismatched or partially matched with the subject into which the implant will be placed (e.g. HLA partial mismatch or MHC partial mismatch). In embodiments where the engineered endothelial cells are derived from an allogeneic donor it may be necessary to treat the subject with one or more immunosuppressive agents in order to reduce the risk of rejection of the "foreign" endothelial cells.

### Kits

The present invention also provides kits for making the implants described herein, and for carrying out the various methods described herein. Such kits may contain any of the components described herein, including, but not limited to, nucleotide sequences (for example encoding E4ORF1), engineered endothelial cells (e.g. E4ORF1+ endothelial cells), naive endothelial cells, means or compositions for detection of engineered endothelial cells or the proteins or nucleic acid molecules expressed therein, (e.g. nucleic acid probes, antibodies, etc.), media or compositions useful for culturing engineered endothelial cells, or for maintaining or culturing implants containing engineered endothelial cells, biocompatible scaffold materials, or any combination thereof. All such kits may optionally comprise instructions for use, containers, culture vessels and the like. A label may accompany the kit and may include any writing or recorded material, which may be electronic or computer readable form (e.g., disk, optical disc, memory chip, or tape) providing instructions or other information for use of the kit contents.

Certain aspects of the present invention may be further described in the following non-limiting Examples.

### EXAMPLES

### Example 1: In Vitro Culture of Implants Containing Engineered Endothelial Cells

Engineered E4ORF1+ human umbilical vein endothelial cells (HUVECs) expressing the adenovirus E4ORF1 protein and green fluorescent protein (GFP) were cultured as described in Example 3. The engineered E4ORF1+ HUVECs were plated under standard conditions in a 24 well plate and were allowed to reach confluence. De-cellularized porcine collagen matrix was used (XenMatrix^{™} Surgical Graft, Bard Davol, Inc. Warwick, RI). The collagen matrix was prepared either by sheering the matrix material into small pieces with scissors or using a 6mm dermal punch. Two alternative methods were used to seed the collagen matrix with the engineered HUVECs. In the first method, the collagen matrix was placed directly on top of monolayers of the engineered HUVECs, which were being cultured on the surface of 24 well plates. In the second method, collagen matrix was placed into wells of a 24 well plate, and engineered HUVECs that had been harvested from their culture vessels were placed on top of the collagen matrix. The engineered HUVECs in both conditions were observed growing into the collagen matrix within 24 hours, and remained viable within the collagen matrix for all time points tested (up to 6 weeks). Blood vessels could be visualized within 48 hours of seeding with the engineered endothelial cells and were present for all time points tested (up to 6 weeks). The engineered E4ORF1+ HUVECs formed blood vessel structures with open lumens, which were observed throughout the collagen matrices (*see* Fig. 1A) and also protruding from the boundaries of the matrices and into the surrounding media (*see* Fig. 1B).

### Example 2: In Vivo Implantation of Collagen Matrices Containing Engineered Endothelial Cells

Collagen matrices were seeded with engineered HUVECs as described in Example 1. Cells were cultured *in vitro* for between 8 days and 6 weeks after seeding with the engineered HUVECs, and then subsequently implanted into mice (immunocompromised mice were used to minimize any rejection reaction to the human endothelial cells). Mice were anesthetized and prepared for surgery. An 8 mm incision was made in the skin of the abdomen. Forceps were inserted between the abdominal muscle and skin to create a space to be occupied by the implants. Each animal received one "control" implant (comprising collagen matrix material that had been soaked in media, but that had not been seeded with endothelial cells) and one "test" implant (comprising the engineered HUVECs). The implants were harvested at 48 hours to 4 weeks following implantation. When removing the implants it was found that the test implants were adhered to the surrounding mouse tissues much more strongly than the control implants. At the 48 hour time point the test implants were already partially anastomosed as evidenced by the presence of blood penetrating the graft (Figure 2A) whereas the control implants were not (Figure 2B). During the removal of the graft, the test matrix was also noticeably adherent to the surrounding tissue whereas the matrix without endothelial cells demonstrated no adherence. Figure 3 illustrates the far greater degree of vascularization seen in the test implants as compared to the controls at 4 weeks following implantation. Implants were sectioned and examined histologically. It appeared that the test implants exhibited a greater degree of cellularity than the control implants. See Figure 4.

### Example 3: Culture of E4ORF 1+ engineered endothelial cells and implants containing E4ORF1+ engineered endothelial cells.

For the purposes of culturing endothelial cells on collagen matrices human umbilical vein endothelial cells (HUVECs) were obtained from consenting donors with no known maladies and transduced with adenovirus E4ORF 1 to generate E4ORF 1+ HUVECs. The engineered HUVECs were grown as adherent cells using methods standard in the art. This standard includes splitting the cells when 100% confluent, culturing at 37°C, expansion on tissue culture treated plates, flasks, or wells, and using a media defined as: Medium 199 supplemented with endothelial cell supplement (50µg/ml), fetal bovine serum (FBS, final concentration: 20%), an antibiotic-antimycotic solution, HEPES buffer (10mM), heparin (50µg/ml), and Glutamax. As the cells proliferated, media was exchanged either during subculturing into larger flasks or if the media exceeded 2 days of culture.

The endothelial cells were only placed into contact with the matrices when the matrices were hydrated. Several variations of the culture method were used when culturing the E4ORF1+ endothelial cells with collagen matrices. In one method, the solid collagen matrix was cut into 25mm² pieces with sharp bladed instruments (such as razor blades, scalpels, or scissors). In another method, the solid collagen matrix was cut into circular pieces with dental punches. In another method the E4ORF1+ endothelial cells (ECs) were in a confluent monolayer with the matrix placed on top with ample media to cover the matrix. The ECs migrated into the matrix. In one method of culturing, the matrix was placed in a well with ample media to cover the matrix. E4ORF1+ ECs were added in a suspension of media to the matrix-containing well. The ECs adhered to and grew on the matrix until contact-mediated growth inhibition. For long term incubations, media was exchanged every 2-3 days with care taken not to directly contact the matrix to prevent disruption of the vascular structures. Invasion of the collagen matrix by the E4ORF1+ ECs was noted within 24 hours in each method with increasing invasion continuing for several days depending on the number of cells seeded and the size of the graft. Overgrowth (defined as E4ORF1+ ECs growing in multiple layers, balls, or clumps), was not noticed - consistent with the cells being growth arrested at confluence. Lumen-containing structures having the appearance of blood vessel were noted at 48 hours and persisted for several weeks (Figure 1B).

*In vivo* experiments were performed by surgically implanting these matrices into immunocompromised NOD scid gamma (NSG) mice - which were incapable of mounting an immune response to the human E4ORF1+ ECs. Anesthetized mice were cleaned, shaven, and stabilized. A small 8mm incision was made in the skin. Blunt ended forceps were inserted while closed, then opened after inserting between the skin and muscle to create a cavity. This was repeated in the opposite direction. Control and test matrices were then placed in these cavities.

## Claims

1. An isolated implant suitable for surgical implantation into a subject comprising: (a) a biocompatible porous scaffold material comprising extracellular matrix molecules, collagen, fibrin and/or laminin, or decellularized animal tissue, and (b) blood vessels disposed within the biocompatible scaffold material, wherein the blood vessels comprise E4ORF1+ engineered endothelial cells, and wherein the implant does not comprise fibroblasts, fibroblast-derived angiogenic factors or fibroblast-derived extracellular matrix components.

2. The isolated implant of claim 1, comprising a network of connected blood vessels; or
wherein the blood vessels comprise capillaries; or
wherein the blood vessels have open lumens; or
wherein one or more of the blood vessels protrude beyond the boundaries of the biocompatible scaffold material.

3. The isolated implant of any preceding claim, wherein the E4ORF1+ engineered endothelial cells are E4ORF1+ ETV2+; or
wherein the engineered endothelial cells express a recombinant ETS family transcription factor; or
wherein the engineered E4ORF1+ endothelial cells are mammalian endothelial cells; or
wherein the engineered E4ORF1+ endothelial cells are human endothelial cells; or
wherein the engineered E4ORF1+ endothelial cells are derived from human umbilical vein endothelial cells (HUVECs); or
wherein the engineered E4ORF1+ endothelial cells are organ-specific endothelial cells; or
wherein the engineered E4ORF1+ endothelial cells are derived from endothelial cells of the subject into which the implant is to be surgically implanted; or
wherein the engineered E4ORF1+ endothelial cells are derived from endothelial cells of an allogeneic donor having the same MHC- or HLA-type as the subject into which the implant is to be surgically implanted; or
wherein the engineered E4ORF1+ endothelial cells are derived from endothelial cells of an allogeneic donor having a partial MHC- or HLA-type match to the subject into which the implant is to be surgically implanted; or
wherein the engineered E4ORF1+ endothelial cells are derived from endothelial cells of an allogenic donor having differences in the MHC- or HLA-type as compared the subject into which the implant is to be surgically implanted.

4. The isolated implant of claim 1, further comprising stem or progenitor cells disposed within the biocompatible scaffold material, and optionally wherein the stem or progenitors cells are selected from the group consisting of hematopoietic stem cells, bone stem cells, muscle stem cells, neural stem cells, hair follicle stem cells, epithelial stem cells, skin stem cells, mesenchymal stem cells, intestinal stem cells, and spermatogonial stem cells.

5. The isolated implant of claim 1, further comprising one or more additional cell types disposed within the biocompatible scaffold material, and optionally wherein the additional cell types are selected from the group consisting of: hematopoietic cells, bone cells, muscle cells, neural cells, pericytes, hair follicle cells, adipose cells, keratinocytes, epithelial cells, skin cells, intestinal cells, and testicular cells.

6. The isolated implant of any preceding claim, wherein the biocompatible scaffold material is solid at 4°C; or
wherein the biocompatible scaffold material is solid at 21°C; or
wherein the biocompatible scaffold material comprises decellularized porcine tissue; or
wherein the biocompatible scaffold material is not Matrigel; or
wherein the biocompatible scaffold material does not comprise hyaluronic acid.

7. The isolated implant of any preceding claim, wherein the implant does not comprise serum; or
wherein the implant does not comprise exogenous growth factors; or
wherein the implant does not comprise exogenous angiogenic factors; or
wherein the implant does not comprise exogenous VEGF; or
wherein the implant does not comprise exogenous FGF; or
wherein the implant does not comprise fibroblasts; or
wherein the implant does not comprise fibroblast-derived angiogenic factors; or
wherein the implant does not comprise fibroblast-derived extracellular matrix components; or
wherein the implant does not comprise micro-carrier beads; or
wherein the implant does not comprise micro-carrier beads coated with an extracellular matrix molecule.

8. A method of preparing an implant suitable for surgical implantation into a subject, the method comprising: culturing a population of engineered E4ORF1+ endothelial cells in contact with a biocompatible porous scaffold material comprising extracellular matrix molecules, collagen, fibrin and/or laminin, or decellularized animal tissue *in vitro* until blood vessels are formed within the biocompatible scaffold material, thereby forming an implant comprising E4ORF1+ blood vessels.

9. The method of claim 8, wherein the culturing is performed until a network of connected blood vessels is formed; or
wherein the culturing is performed until one or more blood vessels protrude beyond the boundaries of the biocompatible scaffold material.

10. The method of claim 8 or 9, wherein the culturing is performed for at least 24 hours; or
wherein the culturing is performed for at least 48 hours; or
wherein the culturing is performed for at least 3 days; or
wherein the culturing is performed for at least 4 days; or
wherein the culturing is performed for at least 5 days; or
wherein the culturing is performed for at least 1 week.

11. The method of any of claims 8 to 10, wherein the engineered E4ORF1+ endothelial cells are E4ORF1+ ETV2+; or
wherein the engineered E4ORF1+ endothelial cells express a recombinant ETS family transcription factor; or
wherein the engineered E4ORF1+ endothelial cells are organ-specific endothelial cells; or
wherein the engineered E4ORF1+ endothelial cells are mammalian endothelial cells; or
wherein the engineered E4ORF1+ endothelial cells are human endothelial cells; or
wherein the engineered E4ORF1+ endothelial cells are derived from endothelial cells of the subject into which the implant is to be surgically implanted; or
wherein the engineered E4ORF1+ endothelial cells are derived from endothelial cells of an allogeneic donor having the same MHC- or HLA-type as the subject into which the implant is to be surgically implanted; or
wherein the engineered E4ORF1+ endothelial cells are derived from endothelial cells of an allogeneic donor having the same MHC- or HLA-type as the subject into which the implant is to be surgically implanted; or
wherein the engineered E4ORF1+ endothelial cells are derived from endothelial cells of an allogeneic donor having a partial MHC- or HLA-type match to the subject into which the implant is to be surgically implanted; or
wherein the engineered E4ORF1+ endothelial cells are derived from endothelial cells of an allogenic donor having differences in the MHC- or HLA-type as compared the subject into which the implant is to be surgically implanted; or
wherein the engineered E4ORF1+ endothelial cells are derived from human umbilical vein endothelial cells (HUVECs).

12. The method of any of claims 8 to 11, wherein the blood vessels comprise capillaries; or
wherein the blood vessels have lumens; or
wherein the biocompatible scaffold material comprises stem or progenitor cells disposed within it, and optionally wherein the stem or progenitors cells are selected from the group consisting of hematopoietic stem cells, bone stem cells, muscle stem cells, neural stem cells, epithelial stem cells, skin stem cells, hair follicle stem cells, mesenchymal stem cells, intestinal stem cells, and spermatogonial stem cells; or
wherein the biocompatible scaffold material comprises one or more additional cell types disposed within it, and optionally wherein the additional cell types are selected from the group consisting of: hematopoietic cells, bone cells, muscle cells, neural cells, pericytes, hair follicle, adipose cells, keratinocytes, epithelial cells, skin cells, intestinal cells, and testicular cells; or wherein the biocompatible scaffold material is solid at 4°C; or wherein the biocompatible scaffold material is solid at 21°C; or
wherein the biocompatible scaffold material is not Matrigel; or
wherein the biocompatible scaffold material does not comprise hyaluronic acid; or
wherein the biocompatible scaffold material comprises decellularized porcine tissue.

13. The method of any of claims 8 to 12, wherein the culturing is performed in the absence of serum; or wherein the culturing is performed in the absence of exogenous growth factors; or
wherein the culturing is performed in the absence exogenous angiogenic factors; or
wherein the culturing is performed in the absence of exogenous VEGF; or
wherein the culturing is performed in the absence of exogenous FGF; or
wherein the culturing is performed in the absence of fibroblasts; or
wherein the culturing is performed in the absence of fibroblast-derived angiogenic factors; or
wherein the culturing is performed in the absence of fibroblast-derived extracellular matrix components; or
wherein the culturing is performed in the absence of micro-carrier beads; or
wherein the culturing is performed in the absence of micro-carrier beads coated with an extracellular matrix molecule.

14. An implant according to any one of claims 1-7 for use in treating a subject.

15. The implant for the use of claim 14, wherein the subject has a tissue defect, and wherein the implant is intended to be surgically implanted into the subject at the site of the tissue defect; or
wherein the engineered E4ORF1+ endothelial cells are organ-specific endothelial cells and wherein in the implant is surgically implanted into the organ from which the organ-specific endothelial cells were derived; or
wherein the subject is a mammalian subject; or
wherein the subject is a human subject; or
wherein the engineered E4ORF1+ endothelial cells in the implant are derived from the subject's own endothelial cells; or
wherein the engineered E4ORF1+ endothelial cells in the implant are derived from endothelial cells of an allogeneic donor having the same MHC- or HLA-type as the subject; or
wherein the engineered E4ORF1+ endothelial cells in the implant are derived from endothelial cells of an allogeneic donor having a partial MHC- or HLA-type match to the subject into which the implant is to be surgically implanted; or
wherein the engineered E4ORF1+ endothelial cells in the implant are derived from endothelial cells of an allogenic donor having differences in the MHC- or HLA-type as compared the subject into which the implant is to be surgically implanted.

## Patentansprüche

1. Isoliertes Implantat, das für eine chirurgische Implantation in ein Individuum geeignet ist, umfassend: (a) ein biokompatibles poröses Gerüstmaterial, das Moleküle extrazellulärer Matrix, Kollagen, Fibrin und/oder Laminin oder dezellularisiertes tierisches Gewebe umfasst, und (b) Blutgefäße, die innerhalb des biokompatiblen Gerüstmaterials angeordnet sind, wobei die Blutgefäße manipulierte E4ORF1+-Endothelzellen umfassen, und wobei das Implantat keine Fibroblasten, von Fibroblasten abgeleitete angiogene Faktoren oder von Fibroblasten abgeleitete Bestandteile extrazellulärer Matrix umfasst.

2. Isoliertes Implantat nach Anspruch 1, das ein Netzwerk aus verbundenen Blutgefäßen umfasst; oder
wobei die Blutgefäße Kapillaren umfassen; oder
wobei die Blutgefäße offene Lumen aufweisen; oder
wobei eines oder mehrere der Blutgefäße über die Ränder des biokompatiblen Gerüstmaterials hinaus vorstehen.

3. Isoliertes Implantat nach einem vorstehenden Anspruch, wobei es sich bei den manipulierten E4ORF1+- Endothelzellen um E4ORF1+ ETV2+ handelt; oder
wobei die manipulierten Endothelzellen einen rekombinanten Transkriptionsfaktor der ETS-Familie exprimieren; oder
wobei es sich bei den manipulierten E4ORF1+-Endothelzellen um Säugetierendothelzellen handelt; oder
wobei es sich bei den manipulierten E4ORF1+-Endothelzellen um humane Endothelzellen handelt; oder
wobei die manipulierten E4ORF1+-Endothelzellen von humanen Nabelvenenendothelzellen (HUVECs) abgeleitet sind; oder
wobei es sich bei den manipulierten E4ORF1+-Endothelzellen um organspezifische Endothelzellen handelt; oder
wobei die manipulierten E4ORF1+-Endothelzellen von Endothelzellen des Individuums abgeleitet sind, dem das Implantat chirurgisch zu implantieren ist; oder
wobei die manipulierten E4ORF1+-Endothelzellen von Endothelzellen eines allogenen Spenders mit dem gleichem MHC- oder HLA-Typ wie das Individuum abgeleitet sind, dem das Implantat chirurgisch zu implantieren ist; oder
wobei die manipulierten E4ORF1+-Endothelzellen von Endothelzellen eines allogenen Spenders mit einer teilweisen MHC- oder HLA-Typübereinstimmung in Bezug auf das Individuum abgeleitet sind, dem das Implantat chirurgisch zu implantieren ist; oder
wobei die manipulierten E4ORF1+-Endothelzellen von Endothelzellen eines allogenen Spenders mit Unterschieden im MHC- oder HLA-Typ gegenüber dem Individuum abgeleitet sind, dem das Implantat chirurgisch zu implantieren ist.

4. Isoliertes Implantat nach Anspruch 1, das ferner Stamm- oder Vorläuferzellen umfasst, die innerhalb des biokompatiblen Gerüstmaterials angeordnet sind, und optional wobei die Stamm- oder Vorläuferzellen aus der Gruppe ausgewählt sind, die aus hämatopoetischen Stammzellen, Knochenstammzellen, Muskelstammzellen, Nervenstammzellen, Haarfollikelstammzellen, Epithelstammzellen, Hautstammzellen, mesenchymalen Stammzellen, intestinalen Stammzellen und Spermatogonienstammzellen besteht.

5. Isoliertes Implantat nach Anspruch 1, das ferner einen oder mehrere zusätzliche Zelltypen umfasst, die innerhalb des biokompatiblen Gerüstmaterials angeordnet sind, und optional wobei die zusätzlichen Zelltypen aus der Gruppe ausgewählt sind, die besteht aus: hämatopoetischen Zellen, Knochenzellen, Muskelzellen, Nervenzellen, Perizyten, Haarfollikelzellen, Fettzellen, Keratinozyten, Epithelzellen, Hautzellen, intestinalen Zellen und Hodenzellen.

6. Isoliertes Implantat nach einem vorstehenden Anspruch, wobei das biokompatible Gerüstmaterial bei 4 °C fest ist; oder
wobei das biokompatible Gerüstmaterial dezellularisiertes Schweinegewebe umfasst; oder
wobei es sich bei dem biokompatiblen Gerüstmaterial nicht um Matrigel handelt; oder
wobei das biokompatible Gerüstmaterial keine Hyaluronsäure umfasst.

7. Isoliertes Implantat nach einem vorstehenden Anspruch, wobei das Implantat kein Serum umfasst; oder
wobei das Implantat keine exogenen Wachstumsfaktoren umfasst; oder
wobei das Implantat keine exogenen angiogenen Faktoren umfasst; oder
wobei das Implantat keinen exogenen VEGF umfasst; oder
wobei das Implantat keinen exogenen FGF umfasst; oder
wobei das Implantat keine Fibroblasten umfasst; oder
wobei das Implantat keine von Fibroblasten abgeleitete angiogene Faktoren umfasst; oder
wobei das Implantat keine von Fibroblasten abgeleiteten Bestandteile extrazellulärer Matrix umfasst; oder
wobei das Implantat keine Mikroträgerkügelchen umfasst; oder
wobei das Implantat keine Mikroträgerkügelchen umfasst, die mit einem Molekül extrazellulärer Matrix beschichtet sind.

8. Verfahren zum Herstellen eines Implantats, das sich für eine chirurgische Implantation in ein Individuum eignet, wobei das Verfahren umfasst: Kultivieren einer Population von manipulierten E4ORF1+-Endothelzellen in Kontakt mit einem biokompatiblen porösen Gerüstmaterial, das Moleküle extrazellulärer Matrix, Kollagen, Fibrin und/oder Laminin oder dezellularisiertes tierisches Gewebe umfasst, *in vitro,* bis Blutgefäße innerhalb des biokompatiblen Gerüstmaterials gebildet werden, wodurch ein Implantat gebildet wird, das E4ORF1+-Blutgefäße umfasst.

9. Verfahren nach Anspruch 8, wobei das Kultivieren durchgeführt wird, bis ein Netzwerk aus verbundenen Blutgefäßen gebildet wird; oder
wobei das Kultivieren durchgeführt wird, bis ein oder mehrere Blutgefäße über die Ränder des biokompatiblen Gerüstmaterials hinaus vorstehen.

10. Verfahren nach Anspruch 8 oder 9, wobei das Kultivieren für zumindest 24 Stunden durchgeführt wird; oder
wobei das Kultivieren für zumindest 48 Stunden durchgeführt wird; oder
wobei das Kultivieren für zumindest 3 Tage durchgeführt wird; oder
wobei das Kultivieren für zumindest 4 Tage durchgeführt wird; oder
wobei das Kultivieren für zumindest 5 Tage durchgeführt wird; oder
wobei das Kultivieren für zumindest 1 Woche durchgeführt wird.

11. Verfahren nach einem der Ansprüche 8 bis 10, wobei es sich bei den manipulierten E4ORF1+-Endothelzellen um E4ORF1+ ETV2+ handelt; oder
wobei die manipulierten E4ORF1+-Endothelzellen einen rekombinanten Transkriptionsfaktor der ETS-Familie exprimieren; oder
wobei es sich bei den manipulierten E4ORF1+-Endothelzellen um organspezifische Endothelzellen handelt; oder
wobei es sich bei den manipulierten E4ORF1+-Endothelzellen um Säugetierendothelzellen handelt; oder
wobei es sich bei den manipulierten E4ORF1+-Endothelzellen um humane Endothelzellen handelt; oder
wobei die manipulierten E4ORF1+-Endothelzellen von Endothelzellen des Individuums abgeleitet sind, dem das Implantat chirurgisch zu implantieren ist; oder
wobei die manipulierten E4ORF1+-Endothelzellen von Endothelzellen eines allogenen Spenders mit dem gleichem MHC- oder HLA-Typ wie das Individuum abgeleitet sind, dem das Implantat chirurgisch zu implantieren ist; oder
wobei die manipulierten E4ORF1+-Endothelzellen von Endothelzellen eines allogenen Spenders mit dem gleichem MHC- oder HLA-Typ wie das Individuum abgeleitet sind, dem das Implantat chirurgisch zu implantieren ist; oder
wobei die manipulierten E4ORF1+-Endothelzellen von Endothelzellen eines allogenen Spenders mit einer teilweisen MHC- oder HLA-Typübereinstimmung in Bezug auf das Individuum abgeleitet sind, dem das Implantat chirurgisch zu implantieren ist; oder
wobei die manipulierten E4ORF1+-Endothelzellen von Endothelzellen eines allogenen Spenders mit Unterschieden im MHC- oder HLA-Typ gegenüber dem Individuum abgeleitet sind, dem das Implantat chirurgisch zu implantieren ist; oder
wobei die manipulierten E4ORF1+-Endothelzellen von humanen Nabelvenenendothelzellen (HUVECs) abgeleitet sind.

12. Verfahren nach einem der Ansprüche 8 bis 11, wobei die Blutgefäße Kapillaren umfassen; oder
wobei die Blutgefäße Lumen aufweisen; oder
wobei das biokompatible Gerüstmaterial Stamm- oder Vorläuferzellen umfasst, die innerhalb dieses angeordnet sind, und optional wobei die Stamm- oder Vorläuferzellen aus der Gruppe ausgewählt sind, die aus hämatopoetischen Stammzellen, Knochenstammzellen, Muskelstammzellen, Nervenstammzellen, Epithelstammzellen, Hautstammzellen, Haarfollikelstammzellen, mesenchymalen Stammzellen, intestinalen Stammzellen und Spermatogonienstammzellen besteht; oder
wobei das biokompatible Gerüstmaterial einen oder mehrere zusätzliche Zelltypen umfasst, die innerhalb dieses angeordnet sind, und optional wobei die zusätzlichen Zelltypen aus der Gruppe ausgewählt sind, die besteht aus: hämatopoetischen Zellen, Knochenzellen, Muskelzellen, Nervenzellen, Perizyten, Haarfollikel, Fettzellen, Keratinozyten, Epithelzellen, Hautzellen, intestinalen Zellen und Hodenzellen; oder wobei das biokompatible Gerüstmaterial bei 4 °C fest ist; oder wobei das biokompatible Gerüstmaterial bei 21 °C fest ist; oder
wobei es sich bei dem biokompatiblen Gerüstmaterial nicht um Matrigel handelt; oder
wobei das biokompatible Gerüstmaterial keine Hyaluronsäure umfasst; oder
wobei das biokompatible Gerüstmaterial dezellularisiertes Schweinegewebe umfasst.

13. Verfahren nach einem der Ansprüche 8 bis 12, wobei das Kultivieren in Abwesenheit von Serum durchgeführt wird; oder wobei das Kultivieren in Abwesenheit von exogenen Wachstumsfaktoren durchgeführt wird; oder
wobei das Kultivieren in Abwesenheit von exogenen angiogenen Faktoren durchgeführt wird; oder
wobei das Kultivieren in Abwesenheit von exogenem VEGF durchgeführt wird; oder
wobei das Kultivieren in Abwesenheit von exogenem FGF durchgeführt wird; oder
wobei das Kultivieren in Abwesenheit von Fibroblasten durchgeführt wird; oder
wobei das Kultivieren in Abwesenheit von von Fibroblasten abgeleiteten angiogenen Faktoren durchgeführt wird; oder
wobei das Kultivieren in Abwesenheit von von Fibroblasten abgeleiteten Bestandteilen extrazellulärer Matrix durchgeführt wird; oder
wobei das Kultivieren in Abwesenheit von Mikroträgerkügelchen durchgeführt wird; oder
wobei das Kultivieren in Abwesenheit von Mikroträgerkügelchen durchgeführt wird, die mit einem Molekül extrazellulärer Matrix beschichtet sind.

14. Implantat nach einem der Ansprüche 1 bis 7 zur Verwendung bei einer Behandlung eines Individuums.

15. Implantat zur Verwendung nach Anspruch 14, wobei das Individuum einen Gewebedefekt aufweist, und wobei das Implantat dem Individuum an der Stelle des Gewebedefekts chirurgisch zu implantieren ist; oder
wobei es sich bei den manipulierten E4ORF1+-Endothelzellen um organspezifische Endothelzellen handelt und wobei in dem Implantat chirurgisch in das Organ implantiert wird, aus dem die organspezifischen Endothelzellen abgeleitet wurden; oder
wobei es sich bei dem Individuum um ein Säugetierindividuum handelt; oder
wobei es sich bei dem Individuum um ein humanes Individuum handelt; oder
wobei die manipulierten E4ORF1+-Endothelzellen in dem Implantat von den eigenen Endothelzellen des Individuums abgeleitet sind; oder
wobei die manipulierten E4ORF1+-Endothelzellen in dem Implantat von Endothelzellen eines allogenen Spenders mit dem gleichem MHC- oder HLA-Typ wie das Individuum abgeleitet sind; oder
wobei die manipulierten E4ORF1+-Endothelzellen in dem Implantat von Endothelzellen eines allogenen Spenders mit einer teilweisen MHC- oder HLA-Typübereinstimmung in Bezug auf das Individuum abgeleitet sind, dem das Implantat chirurgisch zu implantieren ist; oder
wobei die manipulierten E4ORF1+-Endothelzellen in dem Implantat von Endothelzellen eines allogenen Spenders mit Unterschieden im MHC- oder HLA-Typ gegenüber dem Individuum abgeleitet sind, dem das Implantat chirurgisch zu implantieren ist.

## Revendications

1. Implant isolé adapté à une implantation chirurgicale chez un sujet comprenant : (a) un matériau d'échafaudage poreux biocompatible comprenant des molécules de matrice extracellulaire, du collagène, de la fibrine et/ou de la laminine, ou du tissu animal décellularisé , et (b) des vaisseaux sanguins disposés à l'intérieur du matériau d'échafaudage biocompatible, les vaisseaux sanguins comprenant des cellules endothéliales mises au point par génie génétique E4ORF1+, et l'implant ne comprenant pas de fibroblastes, de facteurs angiogéniques dérivés de fibroblastes ou de composants de matrice extracellulaire dérivés de fibroblastes.

2. Implant isolé selon la revendication 1, comprenant un réseau de vaisseaux sanguins connectés ; ou
dans lequel les vaisseaux sanguins comprennent des capillaires ; ou
dans lequel les vaisseaux sanguins ont des lumières ouvertes ; ou
dans lequel un ou plusieurs des vaisseaux sanguins font saillie au-delà des limites du matériau d'échafaudage biocompatible.

3. Implant isolé selon l'une quelconque des revendications précédentes, dans lequel les cellules endothéliales mises au point par génie génétique E4ORF1+ sont EA4ORF1+ ETV2+ ; ou
dans lequel les cellules endothéliales mises au point par génie génétique expriment un facteur de transcription de la famille ETS recombinant ; ou
dans lequel les cellules endothéliales mises au point par génie génétique E4ORF1+ sont des cellules endothéliales de mammifère ; ou
dans lequel les cellules endothéliales mises au point par génie génétique E4ORF1+ sont des cellules endothéliales humaines ; ou
dans lequel les cellules endothéliales mises au point par génie génétique E4ORF1+ sont dérivées de cellules endothéliales de veine ombilicale humaine (HUVEC) ; ou
dans lequel les cellules endothéliales mises au point par génie génétique E4ORF1+ sont des cellules endothéliales spécifiques d'organe ; ou
dans lequel les cellules endothéliales mises au point par génie génétique E4ORF1+ sont dérivées de cellules endothéliales du sujet dans lequel l'implant doit être implanté chirurgicalement ; ou
dans lequel les cellules endothéliales mises au point par génie génétique E4ORF1+ sont dérivées de cellules endothéliales d'un donneur allogénique ayant le même type MHC ou HLA que le sujet dans lequel l'implant doit être implanté chirurgicalement ; ou
dans lequel les cellules endothéliales mises au point par génie génétique E4ORF1+ sont dérivées de cellules endothéliales d'un donneur allogénique ayant une correspondance partielle de type MHC ou HLA avec le sujet dans lequel l'implant doit être implanté chirurgicalement ; ou
dans lequel les cellules endothéliales mises au point par génie génétique E4ORF1+ sont dérivées de cellules endothéliales d'un donneur allogénique ayant des différences de type MHC ou HLA par rapport au sujet dans lequel l'implant doit être implanté chirurgicalement.

4. Implant isolé selon la revendication 1, comprenant en outre des cellules souches ou progénitrices disposées dans le matériau d'échafaudage biocompatible, et éventuellement dans lequel les cellules souches ou progénitrices sont choisies dans le groupe constitué par les cellules souches hématopoïétiques, les cellules souches osseuses, les cellules souches musculaires, les cellules souches neurales, les cellules souches des follicules pileux, les cellules souches épithéliales, les cellules souches de la peau, les cellules souches mésenchymateuses, les cellules souches intestinales et les cellules souches spermatogoniales.

5. Implant isolé selon la revendication 1, comprenant en outre un ou plusieurs types de cellules supplémentaires disposés dans le matériau d'échafaudage biocompatible, et éventuellement dans lequel les types de cellules supplémentaires sont choisis dans le groupe constitué par : les cellules hématopoïétiques, les cellules osseuses, les cellules musculaires, les cellules neurales, les péricytes, les cellules des follicules pileux, les cellules adipeuses, les kératinocytes, les cellules épithéliales, les cellules de la peau, les cellules intestinales et les cellules testiculaires.

6. Implant isolé selon l'une quelconque des revendications précédentes, dans lequel le matériau d'échafaudage biocompatible est solide à 4°C ; ou
dans lequel le matériau d'échafaudage biocompatible est solide à 21°C ; ou
dans lequel le matériau d'échafaudage biocompatible comprend du tissu porcin décellularisé ; ou
dans lequel le matériau d'échafaudage biocompatible n'est pas du Matrigel ; ou
dans lequel le matériau d'échafaudage biocompatible ne comprend pas d'acide hyaluronique.

7. Implant isolé selon l'une quelconque des revendications précédentes, dans lequel l'implant ne comprend pas de sérum ; ou
dans lequel l'implant ne comprend pas de facteurs de croissance exogènes ; ou
dans lequel l'implant ne comprend pas de facteurs angiogéniques exogènes ; ou
dans lequel l'implant ne comprend pas de VEGF exogène ; ou
dans lequel l'implant ne comprend pas de FGF exogène ; ou
dans lequel l'implant ne comprend pas de fibroblastes ; ou
dans lequel l'implant ne comprend pas de facteurs angiogéniques dérivés de fibroblastes ; ou
dans lequel l'implant ne comprend pas de composants de matrice extracellulaire dérivés de fibroblastes ; ou
dans lequel l'implant ne comprend pas de microbilles porteuses ; ou
dans lequel l'implant ne comprend pas de microbilles porteuses revêtues d'une molécule de matrice extracellulaire.

8. Procédé de préparation d'un implant adapté à une implantation chirurgicale chez un sujet, le procédé comprenant : la culture d'une population de cellules endothéliales mises au point par génie génétique E4ORF1+ en contact avec un matériau d'échafaudage poreux biocompatible comprenant des molécules de matrice extracellulaire, du collagène, de la fibrine et/ou de la laminine, ou du tissu animal décellularisé *in vitro* jusqu'à ce que des vaisseaux sanguins soient formés dans le matériau d'échafaudage biocompatible, formant ainsi un implant comprenant des vaisseaux sanguins E4ORF1+.

9. Procédé selon la revendication 8, dans lequel la culture est effectuée jusqu'à ce qu'un réseau de vaisseaux sanguins connectés soit formé ; ou
dans lequel la culture est effectuée jusqu'à ce qu'un ou plusieurs vaisseaux sanguins dépassent des limites du matériau d'échafaudage biocompatible.

10. Procédé selon la revendication 8 ou 9, dans lequel la culture est effectuée pendant au moins 24 heures ; ou
dans lequel la culture est effectuée pendant au moins 48 heures ; ou
dans lequel la culture est effectuée pendant au moins 3 jours ; ou
dans lequel la culture est effectuée pendant au moins 4 jours ; ou
dans lequel la culture est effectuée pendant au moins 5 jours ; ou
dans lequel la culture est effectuée pendant au moins 1 semaine.

11. Procédé selon l'une quelconque des revendications 8 à 10, dans lequel les cellules endothéliales mises au point par génie génétique E4ORF1+ sont E4ORF1+ ETV2+ ; ou
dans lequel les cellules endothéliales mises au point par génie génétique E4ORF1+ expriment un facteur de transcription de la famille ETS recombinant ; ou
dans lequel les cellules endothéliales mises au point par génie génétique E4ORF1+ sont des cellules endothéliales spécifiques d'organe ; ou
dans lequel les cellules endothéliales mises au point par génie génétique E4ORF1+ sont des cellules endothéliales de mammifère ; ou
dans lequel les cellules endothéliales mises au point par génie génétique E4ORF1+ sont des cellules endothéliales humaines ; ou
dans lequel les cellules endothéliales mises au point par génie génétique E4ORF1+ sont dérivées de cellules endothéliales du sujet dans lequel l'implant doit être implanté chirurgicalement ; ou
dans lequel les cellules endothéliales mises au point par génie génétique E4ORF1+ sont dérivées de cellules endothéliales d'un donneur allogénique ayant le même type MHC ou HLA que le sujet dans lequel l'implant doit être implanté chirurgicalement ; ou
dans lequel les cellules endothéliales mises au point par génie génétique E4ORF1+ sont dérivées de cellules endothéliales d'un donneur allogénique ayant le même type MHC ou HLA que le sujet dans lequel l'implant doit être implanté chirurgicalement ; ou
dans lequel les cellules endothéliales mises au point par génie génétique E4ORF1+ sont dérivées de cellules endothéliales d'un donneur allogénique ayant une correspondance partielle de type MHC ou HLA avec le sujet dans lequel l'implant doit être implanté chirurgicalement ; ou
dans lequel les cellules endothéliales mises au point par génie génétique E4ORF1+ sont dérivées de cellules endothéliales d'un donneur allogénique ayant des différences de type MHC ou HLA par rapport au sujet dans lequel l'implant doit être implanté chirurgicalement ; ou
dans lequel les cellules endothéliales mises au point par génie génétique E4ORF1+ sont dérivées de cellules endothéliales de veine ombilicale humaine (HUVEC).

12. Procédé selon l'une quelconque des revendications 8 à 11, dans lequel les vaisseaux sanguins comprennent des capillaires ; ou
dans lequel les vaisseaux sanguins ont des lumières ; ou
dans lequel le matériau d'échafaudage biocompatible comprend des cellules souches ou progénitrices disposées à l'intérieur de celui-ci, et éventuellement dans lequel les cellules souches ou progénitrices sont choisies dans le groupe constitué de cellules souches hématopoïétiques, de cellules souches osseuses, de cellules souches musculaires, de cellules souches neurales, de cellules souches épithéliales, de cellules souches cutanées, de cellules souches de follicules pileux, de cellules souches mésenchymateuses, de cellules souches intestinales et de cellules souches spermatogoniales ; ou
dans lequel le matériau d'échafaudage biocompatible comprend un ou plusieurs types de cellules supplémentaires disposés à l'intérieur de celui-ci, et éventuellement dans lequel les types de cellules supplémentaires sont choisis dans le groupe constitué par : les cellules hématopoïétiques, les cellules osseuses, les cellules musculaires, les cellules neurales, les péricytes, le follicule pileux, les cellules adipeuses, les kératinocytes, les cellules épithéliales, les cellules cutanées, les cellules intestinales et les cellules testiculaires ; ou dans lequel le matériau d'échafaudage biocompatible est solide à 4°C ; ou dans lequel le matériau d'échafaudage biocompatible est solide à 21°C ; ou
dans lequel le matériau d'échafaudage biocompatible n'est pas du Matrigel ; ou
dans lequel le matériau d'échafaudage biocompatible ne comprend pas d'acide hyaluronique ; ou
dans lequel le matériau d'échafaudage biocompatible comprend du tissu porcin décellularisé.

13. Procédé selon l'une quelconque des revendications 8 à 12, dans lequel la culture est réalisée en l'absence de sérum ; ou dans lequel la culture est réalisée en l'absence de facteurs de croissance exogènes ; ou
dans lequel la culture est réalisée en l'absence de facteurs angiogéniques exogènes ; ou
dans lequel la culture est réalisée en l'absence de VEGF exogène ; ou
dans lequel la culture est réalisée en l'absence de FGF exogène ; ou
dans lequel la culture est réalisée en l'absence de fibroblastes ; ou
dans lequel la culture est réalisée en l'absence de facteurs angiogéniques dérivés de fibroblastes ; ou
dans lequel la culture est réalisée en l'absence de composants de matrice extracellulaire dérivés de fibroblastes ; ou
dans lequel la culture est réalisée en l'absence de microbilles porteuses ; ou
dans lequel la culture est réalisée en l'absence de microbilles porteuses revêtues d'une molécule de matrice extracellulaire.

14. Implant selon l'une quelconque des revendications 1 à 7 destiné à être utilisé dans le traitement d'un sujet.

15. Implant destiné à être utilisé selon la revendication 14, dans lequel le sujet présente un défaut tissulaire, et dans lequel l'implant est destiné à être implanté chirurgicalement dans le sujet au niveau du site du défaut tissulaire ; ou
dans lequel les cellules endothéliales mises au point par génie génétique E4ORF1+ sont des cellules endothéliales spécifiques d'organe et dans lequel l'implant est implanté chirurgicalement dans l'organe à partir duquel les cellules endothéliales spécifiques d'organe ont été dérivées ; ou
dans lequel le sujet est un sujet mammifère ; ou
dans lequel le sujet est un sujet humain ; ou
dans lequel les cellules endothéliales mises au point par génie génétique E4ORF1+ dans l'implant sont dérivées des propres cellules endothéliales du sujet ; ou
dans lequel les cellules endothéliales mises au point par génie génétique E4ORF1+ dans l'implant sont dérivées de cellules endothéliales d'un donneur allogénique ayant le même type MHC ou HLA que le sujet ; ou
dans lequel les cellules endothéliales mises au point par génie génétique E4ORF1+ dans l'implant sont dérivées de cellules endothéliales d'un donneur allogénique ayant une correspondance partielle de type MHC ou HLA avec le sujet dans lequel l'implant doit être implanté chirurgicalement ; ou
dans lequel les cellules endothéliales mises au point par génie génétique E4ORF1+ dans l'implant sont dérivées de cellules endothéliales d'un donneur allogénique ayant des différences de type MHC ou HLA par rapport au sujet dans lequel l'implant doit être implanté chirurgicalement.
